# EUROPÄISCHE PATENTANMELDUNG

(11) **EP 1 208 822 A2**
(43) Veröffentlichungstag der Anmeldung: **29.05.2002**
(21) Anmeldenummer: 01127583.1
(22) Anmeldetag: 19.11.2001
(51) Int. Cl.: A61F 5/01

(54) **Vorrichtung zur medizinischen Versorgung von Gelenkverletzungen**

(30) Priorität: 20.11.2000 DE 10057462
(71) Anmelder: Lohmann & Rauscher GmbH & Co. KG, 56579 Rengsdorf (DE)
(72) Erfinder: Jerosch, Jörg, Prof. Dr., 41462 Neuss (DE); Göller, Eric, 53639 Königswinter (DE)
(74) Vertreter: Patentanwälte Leinweber & Zimmermann

(57) **Zusammenfassung**

Die Erfindung betrifft eine Vorrichtung zur medizinischen Versorgung und Prophylaxe von Gelenkverletzungen, mit mindestens einem mindestens zwei gelenkig miteinander verbundene Stabilisierungselemente (12,14) aufweisenden Gelenk (10) und mindestens einem an mindestens einem der Stabilisierungselemente befestigten und an das zu versorgende Gelenk angrenzendes Körperglied zumindest teilweise umlaufenden, vorzugsweise starren Kopplungselement (20,22), wobei die Lage des Kopplungselementes bezüglich der Gelenkachse einstellbar ist.

## Beschreibung

Die Erfindung betrifft eine Vorrichtung zur medizinischen Versorgung von Gelenkverletzungen, mit mindestens einem mindestens zwei gelenkig miteinander verbundene Stabilisierungselemente aufweisenden Gelenk und mindestens einem an mindestens einem der Stabilisierungselemente befestigten und ein an das zu versorgende Gelenk angrenzendes Körperglied zumindest teilweise umlaufenden, vorzugsweise starren Kopplungselement.

Derartige Vorrichtungen werden beispielsweise in Form von sog. Orthesen zur medizinischen Versorgung von Knieverletzungen eingesetzt. Dabei können diese Knieorthesen in der präoperativen und postoperativen Phase sowie in der Rehabilitation und Prophylaxe verwendet werden. Beim Einsatz der bekannten Knieorthesen dienen die gelenkig miteinander verbundenen Stabilisierungselemente zur Rotationsstabilisierung der über das mindestens eine Kopplungselement daran gekoppelten, an das zu versorgende Gelenk angrenzenden Körperglieder bei Beanspruchungen des zu versorgenden Gelenkes. Zu diesem Zweck ist den Stabilisierungselementen bekannter Knieorthesen üblicherweise eine Begrenzungseinrichtung zugeordnet, mit der die Schwenkbewegung des einen Stabilisierungselementes bezüglich dem anderen Stabilisierungselement um die Gelenkachse begrenzt werden kann. Zusammenwirkend mit dem Kopplungselement kann so eine Begrenzung der Flexion und Extension des zu versorgenden Gelenkes erreicht werden.

Bei handelsüblichen Orthesen wird durch Bereitstellung von zwei jeweils zweigelenkig miteinander verbundene Stabilisierungselemente aufweisenden Gelenken an jeweils einer Seite des zu versorgenden Gelenkes (beispielsweise lateral und medial) eine besonders gute Stabilisierung erreicht, wobei die Stabilisierungselemente der beidseits des zu versorgenden Gelenkes angeordneten Gelenke der Vorrichtung üblicherweise über mindestens ein Kopplungselement miteinander verbunden sind. Dabei weisen handelsübliche Orthesen mindestens zwei Kopplungselemente auf, von denen jedes an einem der Stabilisierungselemente befestigt ist, wobei die Kopplungselemente die an das zu versorgende Gelenk angrenzenden Körperglieder auf einander entgegengesetzten Seiten, beispielsweise dorsal und ventral, umlaufen.

Trotz dieser aufwendigen Stabilisierungsmaßnahmen kann es beim Einsatz herkömmlicher Vorrichtungen der eingangs beschriebenen Art noch zu unerwünschten oder schädlichen Beanspruchungen des zu versorgenden Gelenkes kommen.

Angesichts dieser Probleme im Stand der Technik liegt der Erfindung die Aufgabe zugrunde, eine Vorrichtung zur medizinischen Versorgung von Gelenkverletzungen bereitzustellen, mit der unerwünschte Belastungen des zu versorgenden Gelenkes zuverlässig verhindert werden können.

Erfindungsgemäß wird diese Aufgabe durch eine Weiterbildung der bekannten Vorrichtungen gelöst, die im wesentlichen dadurch gekennzeichnet ist, daß die Lage des Kopplungselementes bezüglich der Gelenkachse einstellbar ist.

Diese Erfindung geht auf die Erkenntnis zurück, daß die im Stand der Technik beobachteten Probleme in erster Linie auf die in einigen Fällen mangelhafte Ankopplung der an das zu versorgende Gelenk angrenzenden Körperglieder an die Stabilisierungselemente zurückzuführen sind. Diese mangelhafte Ankopplung hat wiederum zur Folge, daß es noch zu unkontrollierten Bewegungen dieser Körperglieder kommen kann, welche eine unerwünschte Beanspruchung des zu versorgenden Gelenkes verursachen können. Durch die erfindungsgemäße Weiterbildung der bekannten Vorrichtungen kann diese mangelhafte Ankopplung verbessert werden, weil durch die Einstellung der Lage des Kopplungselementes bezüglich der Gelenkachse eine individuelle Anpassung der Vorrichtung an den Körperbau des zu behandelnden Patienten vorgenommen werden kann. Diese Anpassung ermöglicht eine besonders wirkungsvolle Ankopplung der Bewegung der an das zu versorgende Gelenk angrenzenden Körperglieder an die Stabilisierungselemente und mithin eine zuverlässige Vermeidung unerwünschter Belastungen des zu versorgenden Gelenkes.

Wie im Zusammenhang mit aus dem Stand der Technik bekannten Vorrichtungen bereits erläutert, hat es sich im Sinn einer besonders wirkungsvollen Ankopplung der Körperglieder an die erfindungsgemäße Vorrichtung als besonders günstig erwiesen, wenn mindestens zwei Kopplungselemente vorgesehen sind, von denen jedes an einem der Stabilisierungselemente des Gelenkes der Vorrichtung befestigt ist. Diese Ankopplung kann weiter verbessert werden, wenn die Kopplungselemente die an das zu versorgende Gelenk angrenzenden Körperglieder auf einander entgegengesetzten Seiten umlaufen. Bei Einsatz einer erfindungsgemäßen Vorrichtung in Form einer Knieorthese hat es sich als besonders günstig erwiesen, wenn eines der Kopplungselemente den Oberschenkelknochen dorsal umläuft, während ein anderes Kopplungselement die Unterschenkelknochen (Tibia und Fibula) ventral umläuft.

Wenngleich die Einstellung der Lage der Kopplungselemente bezüglich der Gelenkachse auch dann vorgenommen werden kann, wenn die Kopplungselemente unverlierbar an den Stabilisierungselementen befestigt sind und die Einstellung durch eine Verschiebung der Kopplungselemente längs den Stabilisierungselementen in einer senkrecht zur Gelenkachse verlaufenden Richtung vorgenommen wird, hat es sich im Hinblick auf den Erhalt einer besonders guten Anpassung der erfindungsgemäßen Vorrichtung an die jeweiligen Einsatzbedingungen als besonders vorteilhaft erwiesen, wenn mindestens ein Kopplungselement lösbar mit dem mindestens einen Stabilisierungselement verbunden ist. In diesem Fall kann nicht nur die Lage des Kopplungselementes bezüglich des Stabilisierungselementes zur Anpassung an die jeweiligen Einsatzbedingungen eingestellt werden, sondern auch ein Austausch von Kopplungselementen vorgenommen werden. Das kann beispielsweise dann erforderlich werden, wenn die Form des Kopplungselementes keine zufriedenstellende Ankopplung an das entsprechende Körperglied ermöglicht und aus diesem Grund ein anders geformtes Kopplungselement benutzt werden muß.

Wie eingangs bereits im Zusammenhang mit herkömmlichen Vorrichtungen zur medizinischen Versorgung von Gelenkverletzungen erläutert, hat es sich im Hinblick auf den Erhalt einer besonders zuverlässigen Stabilisierung des zu versorgenden Gelenkes als zweckmäßig erwiesen, wenn zwei koaxial zueinander verlaufende Gelenkachsen aufweisende Gelenke vorgesehen sind. Beim Einsatz einer erfindungsgemäßen Vorrichtung in Form einer Knieorthese kann eines dieser Gelenke medial angeordnet sein, während das andere lateral angeordnet ist. Dabei können diese Gelenke über mindestens ein, vorzugsweise zwei beidseits der Gelenkachse angeordnete Kopplungselemente miteinander verbunden sein, wobei diese Verbindung in besonders vorteilhafter Ausgestaltung der Erfindung jeweils lösbar ausgeführt ist. Auch bei den erfindungsgemäßen Vorrichtungen kann mindestens einem der Gelenke eine Begrenzungseinrichtung zugeordnet sein, mit der eine Schwenkbewegung des einen Stabilisierungselementes bezüglich dem anderen Stabilisierungselement um die Gelenkachse begrenzt wird, um so eine Begrenzung von Flexion und Extension der über die Kopplungselemente an die Gelenke gekoppelten Körperglieder zu erreichen.

Zusätzlich zu den bislang erläuterten Elementen kann eine erfindungsgemäße Vorrichtung auch noch weitere funktionale Elemente, wie etwa eine das zu versorgende Gelenk und/oder mindestens ein an das zu versorgende Gelenk angrenzendes Körperglied umlaufende, vorzugsweise lösbar mit einem der Stabilisierungselemente und/oder dem mindestens einen Kopplungselement verbundene Kompressionseinrichtung, aufweisen. Eine beispielsweise in Form eines Bandagenelementes vorliegende Kompressionseinrichtung kann eingesetzt werden, um Schwellungen entgegenzuwirken und/oder eine Stimulanz der Sensomotorik im Bereich des zu versorgenden Gelenkes herbeizuführen. Im Sinne einer besonders günstigen Anpassung an die jeweiligen Einsatzbedingungen kann die Kompressionseinrichtung zwei oder mehr vorzugsweise lösbar miteinander verbundene Kompressionselemente aufweisen. Dabei kann die lösbare Verbindung in besonders vorteilhafter Ausgestaltung der Erfindung mit Hilfe von Flächenhaftverschlüssen hergestellt werden. Bei einer insbesondere für die Versorgung von Knieverletzungen einsetzbaren Vorrichtung weist die Kompressionseinrichtung neben einem Bandagenkörper auch noch mindestens einen Geleinsatz auf, welcher sich besonders gut an die jeweilige Gelenkform anpaßt. Mit einem derartigen Geleinsatz kann beispielsweise die Patella stabilisiert und einer Patellalateralisation entgegengewirkt werden.

Der Bandagenkörper einer erfindungsgemäßen Vorrichtung kann beispielsweise in Form eines Textillaminates vorliegen.

Gemäß einem weiteren Gesichtspunkt der Erfindung kann eine besonders gute Anpassung einer erfindungsgemäßen Vorrichtung mit mindestens einem mindestens zwei gelenkig miteinander verbundene Stabilisierungselemente aufweisenden Gelenk, mindestens einem mit mindestens einem Stabilisierungselement verbundenen Kopplungselement und mindestens einer mit dem Gelenk und/oder dem Kopplungselement verbundenen Kompressionseinrichtung an die jeweilige Benutzungsbedingungen erreicht werden, wenn alle Verbindungen zwischen dem Stabilisierungselement, dem Kopplungselement und der Kompressionseinrichtung lösbar sind, weil durch die so erreichte Modularität der Gesamtvorrichtung sämtliche Elemente zum Erhalt einer idealen Anpassung an die jeweiligen Einsatzbedingungen ausgetauscht werden können. Im Sinne einer besonders einfachen Anpassung an die jeweiligen Einsatzbedingungen hat es sich als besonders günstig erwiesen, wenn mindestens ein Kopplungselement zumindest teilweise aus thermoplastischem Kunststoff besteht. Zusätzlich oder teilweise kann das Kopplungselement aber auch Teile aus faserverstärktem Kunststoff aufweisen, um so eine besonders hohe Stabilität zu erhalten. Das Stabilisierungselement der erfindungsgemäßen Vorrichtung kann aus Aluminium, Carbon und/oder Glasfasern bestehen. Dabei können die Stabilisierungselemente der erfindungsgemäßen Vorrichtung in besonders vorteilhafter Ausgestaltung anatomisch vorgeformt sein.

Nachstehend wird die Erfindung unter Bezugnahme auf die Zeichnung, auf die hinsichtlich aller erfindungswesentlichen und in der Beschreibung nicht besonders hervorgehobenen Einzelheiten verwiesen wird, erläutert. In der Zeichnung zeigt:
- Fig. 1: eine schematische Seitenansicht einer erfindungsgemäßen Vorrichtung,
- Fig. 2: eine erfindungsgemäße Knieorthese mit modularem Aufbau,
- Fig. 3: einen Geleinsatz für die Knieorthese nach Fig. 1 und
- Fig. 4: schematisch ein Detail der Vorrichtung nach Fig. 1.

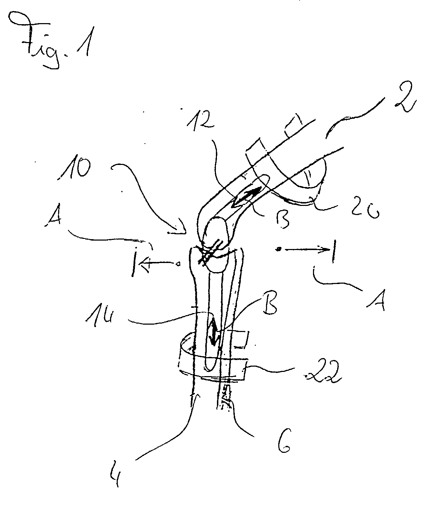

Die in Fig. 1 dargestellte Vorrichtung ist zur postoperativen Behandlung bei Verletzung des vorderen Kreuzbandes gedacht. Diese Vorrichtung umfaßt zwei Gelenke 10, von denen in Fig. 1 jedoch nur eines dargestellt ist, sowie zwei Kopplungselemente 20 und 22. Die Gelenke 10 sind medial und lateral angeordnet und über die Kopplungselemente 20 und 22 miteinander verbunden. Jedes der Gelenke 10 umfaßt zwei in Form von Gelenkschienen 12 und 14 gebildete Stabilisierungselemente, von denen die Gelenkschiene 12 etwa parallel zum Oberschenkelknochen 2 verläuft, während die Gelenkschiene 14 etwa parallel zu den Unterschenkelknochen (Tibia 4 und Fibula 6) verläuft. Das Kopplungselement 14 umläuft den Oberschenkel dorsal, während die Gelenkschiene 16 die Unterschenkelknochen ventral umläuft. Mit der in Fig. 1 dargestellten Anordnung können die in Fig. 1 durch die Symbole A dargestellten Bewegungen nach Behandlungen von Verletzungen des vorderen Kreuzbandes 8 verhindert werden. Dabei kann die Vorrichtung an die jeweiligen Einsatzbedingungen angepaßt werden, indem die Kopplungselemente 20 und 22 längs den Gelenkschienen 12 bzw. 14 verschoben und in der gewünschten Stellung arretiert werden, wie durch die Doppelpfeile B in Fig. 1 dargestellt.

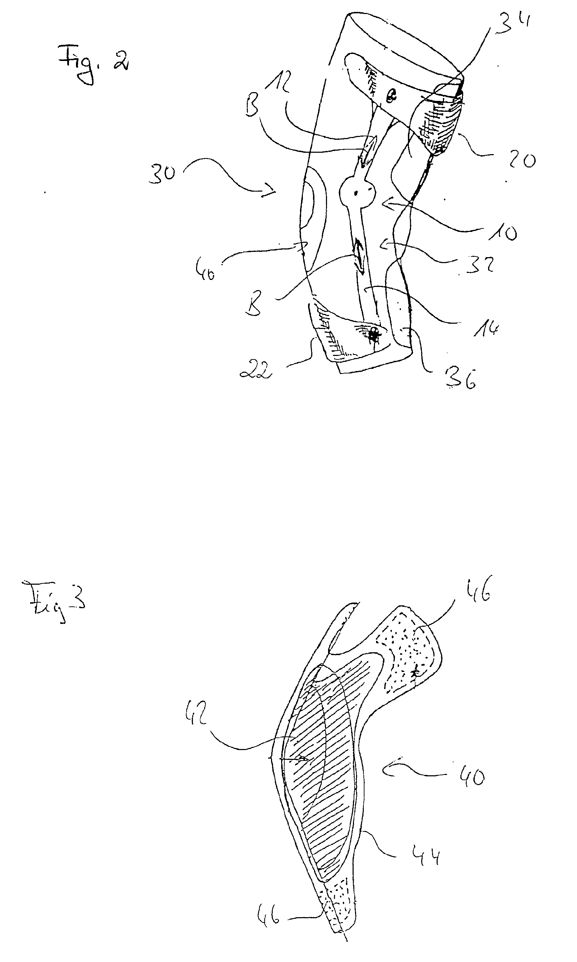

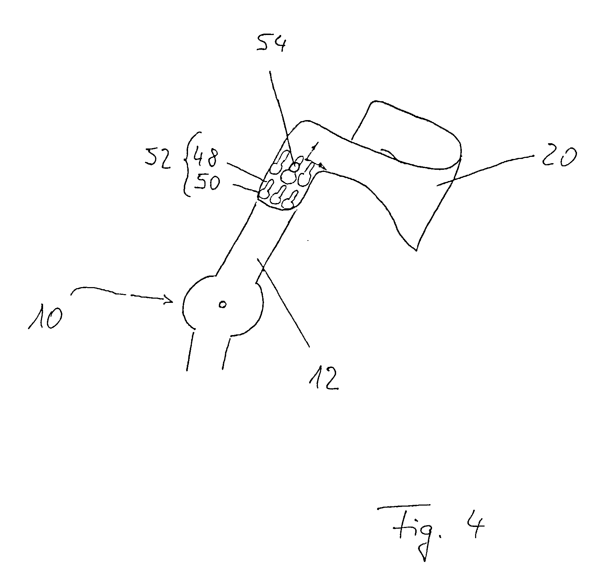

Die in Fig. 2 dargestellte Ausführungsform der Erfindung weist neben den anhand der Fig. 1 bereits erläuterten Elemente auch noch eine insgesamt mit 30 bezeichnete Kompressionseinrichtung auf, die lösbar an den Gelenkschienen 12 und 14 befestigt ist. Dazu können Flächenhaftverschlüsse oder in der Kompressionseinrichtung vorgesehene Taschen zur Aufnahme der Gelenkschienen eingesetzt werden. Die Kompressionseinrichtung weist einen Bandagenkörper 32 sowie einen insgesamt mit 40 bezeichneten Geleinsatz auf, der im Bereich der Kniescheibe am Kniegelenk anliegt. Der Bandagenkörper 32 umläuft die an das Kniegelenk angrenzenden Teile des Oberschenkels und Unterschenkels und kann mit Hilfe von an seinen Rändern vorgesehenen Flächenhaftverschlüssen 34 bzw. 36 festgelegt werden. Der in Fig. 3 dargestellte Geleinsatz umfaßt ein Gelkissen 42 sowie einen das Gelkissen 42 haltenden Textilstreifen 44. Im Bereich des oberen und unteren Randes des Textilstreifens 44 sind Flächenhaftverschlüsse 46 vorgesehen, mit denen der Geleinsatz am Bandagenkörper 32 festgelegt werden kann.

Wie in Fig. 2 besonders deutlich zu erkennen ist, sind die aus einem thermoplastischen Kunststoff hergestellten Kupplungselemente 20 und 22 anatomisch geformt und weisen eine flächige Anlagefläche für die an das Kniegelenk angrenzenden Körperglieder auf. Die in der Zeichnung dargestellte Ausführungsform ist insgesamt modular aufgebaut, so daß die Gelenke 10, Kupplungselemente 20 und 22 sowie die Kompressionseinrichtung 30 lösbar miteinander verbunden sind. Daher können sämtliche Einzelteile der in Fig. 2 dargestellten Vorrichtung zur Anpassung an die jeweiligen Einsatzbedingungen ausgetauscht werden. Darüber hinaus ist auch noch die Kompressionseinrichtung 30 modular aufgebaut, so daß der Geleinsatz und der Bandagenkörper je nach Einsatzgebiet ausgetauscht werden können.

Fig. 4 zeigt schematisch eine Möglichkeit der Realisierung der Verschiebbarkeit der Kopplungselemente 20 und 22 längs der Gelenkschienen 12 bzw. 14, die in den Fig. 1 und 2 durch die Doppelpfeile B angedeutet ist. Dazu sind Langlöcher, beispielsweise an dem Kopplungselement 20, vorgesehen, die einen schmaleren Abschnitt 48 und einen weiteren Abschnitt 50 aufweisen. Die Langlöcher sind insgesamt mit der Bezugszahl 52 bezeichnet. Beispielsweise an der Gelenkschiene 12 ist passend dazu ein Einrastknopf 54 vorgesehen, der pilzförmige Struktur hat. Dabei paßt der Stamm des Einrastknopfes 54 durch die Langlöcher 52, und zwar sowohl in dem schmalen Abschnitt 48 als auch in dem weiten Abschnitt 50. Der Kopf des Einrastknopfes 54 paßt hingegen nur durch den weiten Abschnitt 50, nicht aber durch den schmalen Abschnitt 48 der Langlöcher 52. In angebrachtem Zustand sind dadurch die Kopplungselemente 20 und 22 längs den Gelenkschienen 12 bzw. 14 verschieblich, indem der Einrastknopf 54 längs dem schmalen Abschnitt 48 des Langlochs 52 verschoben wird. Der Einrastknopf 54 kann als Schraube ausgestaltet sein, die in ein entsprechendes Gewinde in der Gelenkschiene 12 bzw. 14 eingeschraubt wird, wodurch eine Arretierung des Kopplungselementes 20 bzw. 22 in der gewünschten Relativstellung zu der Gelenkschiene 12 bzw. 14 möglich ist, weil die Unterseite des Kopfes des Einrastknopfes einen das Langloch 52 umgebenden Bereich des Kopplungselementes 20 bzw. 22 festklemmt.

Wie vorstehend erläutert, kann die anhand der Fig. 1 bis 4 dargestellte Ausführungsform der Erfindung beispielsweise zur postoperativen Behandlung von Verletzungen des vorderen Kreuzbandes eingesetzt werden. Daneben können Vorrichtungen der in den Fig. 1 bis 4 dargestellten Art aber auch zur posttraumatischen und postoperativen Versorgung anderer Knieverletzungen, im Rahmen der Rehabilitation sowie zur Rezidivprophylaxe eingesetzt werden, was nur durch den modularen Aufbau ermöglicht wird. Im übrigen ist die Erfindung auch nicht auf die in der Zeichnung dargestellten Knieorthesen beschränkt. Vielmehr kann die Grundidee des modularen Aufbaus und der variablen Anbringung einzelner Vorrichtungselemente auch für Therapien und Prophylaxen bei anderen Gelenken, wie etwa Fußgelenken, eingesetzt werden, weil die Grundidee des modularen Aufbaus die Therapieprinzipien aller Ligamentverletzungen der Gelenke berücksichtigt. Dazu muß das Produkt lediglich den anatomischen Erfordernissen angepaßt werden.

## Patentansprüche

1. Vorrichtung zur medizinischen Versorgung und Prophylaxe von Gelenkverletzungen, mit mindestens einem mindestens zwei gelenkig miteinander verbundene Stabilisierungselemente (12, 14) aufweisenden Gelenk (10) und mindestens einem an mindestens einem der Stabilisierungselemente (12, 14) befestigten und ein an das zu versorgende Gelenk angrenzendes Körperglied zumindest teilweise umlaufenden, vorzugsweise starren Kopplungselement (20, 22), **dadurch gekennzeichnet, daß** die Lage des mindestens einen Kopplungselementes (20, 22) bezüglich der Gelenkachse einstellbar ist.

2. Vorrichtung nach Anspruch 1, **gekennzeichnet durch** mindestens zwei Kopplungselemente (20, 22), von denen jedes an einem der Stabilisierungselemente (12, 14) befestigt ist, wobei die Kopplungselemente (20, 22) die an das zu versorgende Gelenk angrenzenden Körperglieder auf einander entgegengesetzten Seiten umlaufen.

3. Vorrichtung nach Anspruch 1 oder 2, **dadurch gekennzeichnet, daß** mindestens ein Kopplungselement (20, 22) lösbar mit dem mindestens einen Stabilisierungselement (12, 14) verbunden ist.

4. Vorrichtung nach einem der vorhergehenden Ansprüche, **gekennzeichnet durch** mindestens zwei koaxial zueinander verlaufende Gelenkachsen aufweisende Gelenke.

5. Vorrichtung nach Anspruch 4, **dadurch gekennzeichnet, daß** die Gelenke über mindestens ein Kopplungselement (20, 22) miteinander verbunden sind.

6. Vorrichtung nach einem der vorhergehenden Ansprüche, **dadurch gekennzeichnet, daß** das mindestens eine Gelenk eine Begrenzungseinrichtung zur Begrenzung der Schwenkbewegung des einen Stabilisierungselementes (12) bezüglich dem anderen Stabilisierungselement (14) um die Gelenkachse aufweist.

7. Vorrichtung nach einem der vorhergehenden Ansprüche, **gekennzeichnet durch** mindestens eine das zu versorgende Gelenk und/oder mindestens ein an das zu versorgende Gelenk angrenzendes Körperglied umlaufende, vorzugsweise lösbar mit mindestens einem der Stabilisierungselemente (12, 14) und/oder dem mindestens einen Kopplungselement (20, 22) verbundene Kompressionseinrichtung (30).

8. Vorrichtung nach Anspruch 7, **dadurch gekennzeichnet, daß** die Kompressionseinrichtung (30) mindestens zwei vorzugsweise lösbar miteinander verbundene Kompressionselemente (32, 40) aufweist.

9. Vorrichtung nach Anspruch 7 oder 8, **dadurch gekennzeichnet, daß** die Kompressionseinrichtung (30) mindestens einen Bandagenkörper (32) und/oder mindestens einen Geleinsatz (40) aufweist.

10. Vorrichtung nach Anspruch 9, **dadurch gekennzeichnet, daß** der Bandagenkörper (32) ein Textillaminat aufweist.

11. Vorrichtung, insbesondere nach einem der Ansprüche 7 bis 9, mit mindestens einem mindestens zwei gelenkig miteinander verbundene Stabilisierungselemente (12, 14) aufweisenden Gelenk, mindestens einem mit mindestens einem Stabilisierungselement (12, 14) verbundenen Kopplungselement (20, 22) und mindestens einer mit dem Gelenk (10) und/oder dem Kopplungselement (20, 22) verbundenen Kompressionseinrichtung (30), **dadurch gekennzeichnet, daß** alle Verbindungen zwischen dem Stabilisierungselement (12, 14), dem Kopplungselement (20, 22) und der Kompressionseinrichtung (30) lösbar sind.

12. Vorrichtung nach einem der vorhergehenden Ansprüche, **dadurch gekennzeichnet, daß** mindestens ein Kopplungselement (20, 22) zumindest teilweise aus thermoplastischem Kunststoff und/oder faserverstärktem Kunststoff besteht.

13. Vorrichtung nach einem der vorhergehenden Ansprüche, **dadurch gekennzeichnet, daß** mindestens ein Stabilisierungselement zumindest teilweise aus Aluminium, Carbon und/oder Glasfasern besteht.
